# EUROPEAN PATENT SPECIFICATION

(11) **EP 3 881 681 B1**
(45) Date of publication and mention of the grant of the patent: **01.11.2023**
(21) Application number: 19885058.8
(22) Date of filing: 13.09.2019
(51) Int. Cl.: A23F 3/16, A23F 5/24, A23L 2/52, A23L 2/70, A23L 2/62, A23L 2/02, A23L 2/06

(54) **CHITOSAN-CONTAINING BEVERAGE**
CHITOSANHALTIGES GETRÄNK
BOISSON CONTENANT DU CHITOSANE

(30) Priority: 14.11.2018 JP 2018214070
(43) Date of publication of application: 22.09.2021
(73) Proprietor: Suntory Holdings Limited, Osaka 530-8203 (JP)
(72) Inventor: SHIMADA, Yoshiaki, Kawasaki-shi, Kanagawa 211-0067 (JP); YOSHIDA, Atsushi, Kawasaki-shi, Kanagawa 211-0067 (JP); OSADA, Tomoya, Tokyo 104-0031 (JP); TAMURA, Hiroshi, Suita-shi, Osaka 564-8680 (JP); FURUIKE, Tetsuya, Suita-shi, Osaka 564-8680 (JP); KOMOTO, Daiki, Suita-shi, Osaka 564-8680 (JP)
(74) Representative: Vossius & Partner Patentanwälte Rechtsanwälte mbB
(86) International application number: PCT/JP2019/036080
(87) International publication number: WO 2020/100405

(56) References cited:
- JP-A- H 067 117
- JP-A- H07 111 857
- JP-A- H07 236 419
- JP-A- 2001 316 271
- JP-A- 2005 075 957
- JP-A- 2005 328 844
- JP-A- 2007 110 982
- JP-A- 2019 115 325
- US-A1- 2005 196 497
- US-A1- 2005 196 497
- MARTÍ NURIA ET AL: "Vitamin C and the role of citrus juices as functional food", NATURAL PRODUCT COMMUNICATIONS, vol. 4, no. 5, 1 May 2009 (2009-05-01), pages 677-700, XP002806975, ISSN: 1934-578X, DOI: 10.1177/1934578x0900400506
- HIROKAZU OKUMA ET AL: "Determination of L-ascorbic acid in green tea with an enzyme reactor electrode system", JOURNAL OF JAPANESE SOCIETY OF FOOD SCIENCE AND TECHNOLOGY, [Online] vol. 43, no. 6, 15 June 1996 (1996-06-15), pages 668-673, XP055814331, JP ISSN: 1341-027X, DOI: 10.3136/nskkk.43.668 Retrieved from the Internet: URL:https://www.jstage.jst.go.jp/article/n skkk1995/43/6/43_6_668/_article/-char/en> [retrieved on 2022-07-01]
- Anonymous: "Reference Daily Intakes (RDIs) in the New Nutrition Facts Label | FDA", , 15 September 2018 (2018-09-15), pages 1-3, XP55937423, Retrieved from the Internet: URL:https://www.fda.gov/media/99069/downlo ad [retrieved on 2022-07-01]
- Hirokazu Okuma , Hitoshi Takahashi , Seiichi Yazawa , Shizuko Horike , Ryoichi Akahoshi: "Determination of L-ascorbic acid in green tea with an enzyme reactor electrode system", Journal of the Japanese Society for Food Science and Technology, vol. 43, no. 6, 15 June 1996 (1996-06-15), pages 668-673, XP055814331, ISSN: 1341-027X, DOI: 10.3136/nskkk.43.668

## Description

### TECHNICAL FIELD

The present invention relates to a chitosan-containing beverage. More specifically, the present invention relates to a beverage composition containing specific amounts of chitosan and vitamin C, which is prevented from clouding.

### BACKGROUND ART

In recent years, various functions of chitosan have been clarified. For example, an effect on promoting calcium absorption (NPL 1) and an effect of anti-obesity (NPL 2) are known as the functions of chitosar. NPL 3 discloses an orange juice from Valencia Late from Spanish origin comprising 41,9 mg/100 mL (419 ppm) of vitamin C. NPL 4 discloses a green tea extract which comprises 13.5 to 29 mg/100 mL (135 to 290 ppm) of vitamin C. NPL 5 discloses that the RDI of vitamin C for adults and children over 4 years of age is 90 mg.

Various suggestions have been made for adding chitosan to foods and beverages. For example, PTL 1 suggests that addition of chitosan to a weakly acidic beverage having a pH of 4.6 or more prevents decrease in pH. PTL 2 discloses that by combining chitosan with chondroitin sulfuric acid, a beverage with a suppressed bitter taste of chitosan is obtained. Furthermore, PTL 3 discloses that by using chitosan having a particular molecular weight distribution, a chitosan-containing beverage that is stable even in neutral to alkaline regions is obtained. PTL 4 discloses a fruit juice-containing beverage comprising chitosan, wherein the concentration of chitosan in the beverage is about 10 to about 50 ppm.

### CITATION LIST

### PATENT LITERATURES

PTL 1: Japanese Patent Laid-Open No. 2001-000158
PTL 2: Japanese Patent Laid-Open No. 2009-055882
PTL 3: Japanese Patent Laid-Open No. 2005-075957
PTL4: US 2005/196497 A1

### NON PATENT LITERATURES

NPL 1: Chitin and Chitosan Research Vol. 4, No. 2, 1998, pp. 170-171
NPL 2: Chitin and Chitosan Research Vol. 4, No. 2, 1998, pp. 166-167
NPL 3: MARTI NURIA ET AL: "Vitamin C and the role of citrus juices as functional food", NATURAL PRODUCT COMMUNICATIONS, vol. 4, no. 5, 1 May 2009, pages 677-700
NPL 4: HIROKAZU OKUMA ET AL: "Determination of L-ascorbic acid in green tea with an enzyme reactor electrode system",JOURNAL OF JAPANESE SOCIETY OF FOOD SCIENCE AND TECHNOLOGY, [Online] vol. 43, no. 6, 15 June 1996, pages 668-673
NPL 5: Anonymous: "Reference Daily Intakes (RDIs) in the New Nutrition Facts Label | FDA",15 September 2018, pages 1-3

### SUMMARY OF INVENTION

### TECHNICAL PROBLEM

The present inventors have conducted various studies on chitosan-containing beverages, and found that it is not only necessary to disperse the chitosan uniformly but also is necessary to prevent occurrence of cloudiness. In beverages such as containerized beverages, it may take time from the manufacture to the consumption of the beverages. If occurrence of cloudiness is not prevented, an amount of intake of chitosan becomes uneven when drinking, and the appearance of the beverage becomes undesirable.

In view of these circumstances, an object of the present invention is to prevent a chitosan-containing beverage from clouding.

### SOLUTION TO PROBLEM

The present inventors have intensively studied to solve the problems, and have found that when a naturally derived material, chitosan, is added to a beverage, the occurrence of cloudiness due to chitosan is prevented by combining with vitamin C (ascorbic acid). The present inventors have further investigated and found that the combination of chitosan and vitamin C in specific amounts is important for preventing the beverage from clouding, and have completed the present invention.

### ADVANTAGEOUS EFFECTS OF INVENTION

According to the present invention, the occurrence of cloudiness can be effectively prevented in a chitosan-containing beverage.

### BRIEF DESCRIPTION OF DRAWINGS

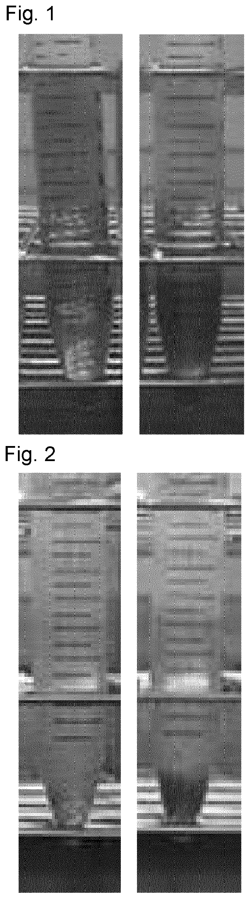

Fig. 1 is a photograph showing cloudiness occurring in an oolong tea beverage (left: no occurrence of cloudiness, right: occurrence of cloudiness).
Fig. 2 is a photograph showing cloudiness occurring in a green tea beverage (left: no occurrence of cloudiness, right: occurrence of cloudiness).

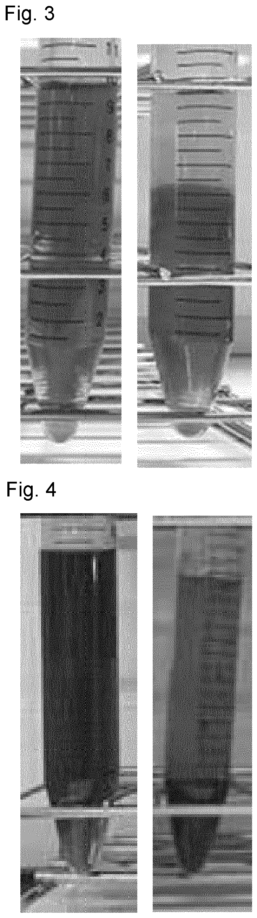

Fig. 3 is a photograph showing cloudiness occurring in a black tea beverage (left: no occurrence of cloudiness, right: occurrence of cloudiness).
Fig. 4 is a photograph showing cloudiness occurring in a coffee beverage (left: no occurrence of cloudiness, right: occurrence of cloudiness).

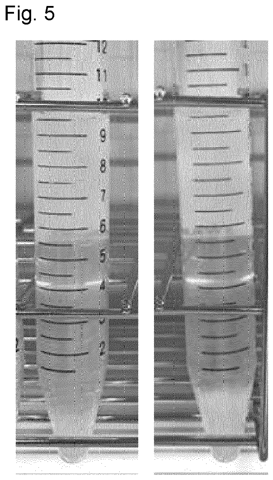

Fig. 5 is a photograph showing cloudiness occurring in a fruit juice-containing beverage (left: no occurrence of cloudiness, right: occurrence of cloudiness).

### DESCRIPTION OF EMBODIMENTS

The present invention relates to a beverage containing chitosan and vitamin C in specific amounts.

### Chitosan

The beverage of the present invention contains chitosan. Chitosan is a linear polysaccharide and is a 1,4-polymer of glucosamine. The molecular formula of chitosan is (C₆H₁₁NO₄)ₙ, and the molecular weight may reach hundreds of thousands depending on the polymerization degree. As used herein, the 1,4-polymer of glucosamine is referred to as chitosan.

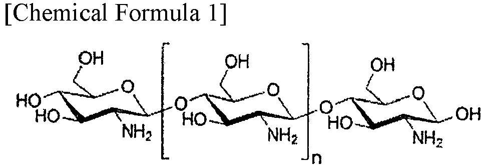

The molecular weight of the chitosan is 100 to 1000 kDa, such as for example, 900 kDa or less, more preferably 800 kDa or less, and further preferably 700 kDa or less. In one embodiment, the chitosan has a molecular weight of 300 kDa or more. The chitosan may be used in the form of a free amine or as a salt with an appropriate acid. The salt form is not particularly limited as long as it is a salt that can be used for food. Examples thereof include salts with organic acids such as an acetate salt, a lactate salt and a citrate salt, and salts with inorganic acids such as a hydrochloride salt and a sulfate salt. Preferred salts are hydrochloride salts.

Chitosan is generally difficult to dissolve in water, depending on its polymerization degree, and especially difficult to dissolve in neutral to alkaline solutions. Thus, when adding chitosan to a containerized beverage or the like, not only adding chitosan to the solution uniformly but also preventing occurrence of cloudiness, precipitation, or the like over time becomes technical hurdles. In addition, the ingredients contained in the beverage may associate with chitosan, leading to a tendency of occurring cloudiness or precipitation. Thus, it is necessary to develop technology according to the type of beverage to which the chitosan is added, in order to produce a chitosan-containing beverage having excellent stability. In the present invention, by adding chitosan to a beverage in combination with vitamin C in a specific proportion, a beverage having excellent stability can be obtained.

Chitosan can be obtained by deacetylation of chitin obtained mainly from the exoskeleton of crustaceans such as crab and shrimp. Conversion of chitin to chitosan (deacetylation reaction) sometimes does not proceed completely and chitosan may contain N-acetylglucosamine partially on the carbohydrate chain. In commercially available chitosan products, a percentage of deacetylation (%DA) is often indicated. The %DA of commercially available chitosan is mostly 60 to 100%.

In the present invention, the amount of chitosan added to the beverage is 1 to 75 ppm. Chitosan is substantially harmless as a food product, and is nearly tasteless and odorless. However, when the amount of chitosan added exceeds 100 ppm, occurrence of cloudiness caused by chitosan and the like becomes prominent in the beverage even when chitosan is used in combination with vitamin C. In a preferred embodiment, the concentration of chitosan in the beverage of the present invention may be 2 to 65 ppm or 5 to 55 ppm.

In the present invention, chitosan can be added in an appropriate step in the production of a variety of beverages. Examples of the addition method include a method of adding chitosan to a raw material in advance, a method of adding chitosan in the process of formulating raw material ingredients, and a method of adding chitosan after formulated ingredients were dissolved into water. When sterilization of the beverage is performed, chitosan may be added prior to or after the sterilization.

### Vitamin C (ascorbic acid)

The beverage of the present invention contains vitamin C. Vitamin C is a water-soluble vitamin, and also referred to as L-ascorbic acid. Vitamin C has excellent reducibility and is often used in foods as an antioxidant.

In the present invention, the amount of vitamin C added to the beverage is 1 to 650 ppm. When the amount of vitamin C added is high, cloudiness caused by chitosan may be occurred in the beverage. In a preferred embodiment, the concentration of vitamin C in the beverage of the present invention is 5 to 600 ppm, further preferably 10 to 550 ppm, and still more preferably 50 to 500 ppm.

The amount of vitamin C added may be adjusted by adding a raw material containing vitamin C to a beverage, or by adding vitamin C in the form of a synthesized reagent or a reagent extracted from natural sources.

### Beverage

The present invention relates to a beverage. In a preferred embodiment, the beverage of the present invention is a beverage that is not likely to have precipitation even after long-term storage at room temperature. In other words, precipitation over time is prevented in the beverage of the present invention, and the appearance of the beverage can be maintained uniformly over a long period of time, and thus the beverage of the present invention is suitably provided as a containerized beverage. The "containerized beverage" herein refers to a beverage that is contained in a resin container such as a polyethylene terephthalate container, a can, a bottle, a paper container, or the like. Since precipitation over time can be prevented in the beverage of the present invention, it is preferable, in one embodiment, that the beverage be a containerized beverage filled in a transparent container.

### (Tea Beverage)

In one embodiment, the beverage of the present invention relates to a tea beverage. The tea beverage of the present invention can be obtained by adding an extract of tea. The extract of tea is obtained by extracting raw material tea leaves with warm water and removing extraction residues from the extract of tea. In the tea beverage of the present invention, a tea extract solution obtained from a leaf of a tree belonging to the genus Camellia sinensis, a plant of genus Camellia, or a processed product thereof is added. As the tea, any of a fermented tea, a semi-fermented tea, or a non-fermented tea can be used without limitation. The cultivar, production area, harvest time, harvest method, cultivation method, and the like of the raw material tea leaves are not limited. For example, raw tea leaves including leaves and stems can be used as the raw material tea leaves. Preferred examples of the non-fermented tea include green teas such as sencha, gyokuro, and tencha. Preferred examples of the semi-fermented tea include oolong tea (blue tea) such as Tieguanyin and Huangjin Gui. Preferred examples of the fermented tea include black tea.

Extraction can be performed by known methods using, for example, an extraction apparatus such as a kneader. Specifically, the extraction may be performed under normal pressure or under increased pressure with extraction water at 60 to 100°C (preferably at 70 to 90°C) in the amount 20 to 100 times relative to raw material tea leaves for about 1 minute to 20 minutes, if necessary, with stirring once to several times. Examples of the extraction water used for extraction include pure water (including hard water, soft water, and ion exchange water), aqueous vitamin C-containing solution, and pH-adjusted water. During or after the extraction, an antioxidant such as vitamin C or a pH adjusting agent such as sodium hydrogen carbonate may be added to the tea extract solution.

The tea extract solution obtained by extracting raw material tea leaves may then be subjected to filtration or the like to remove extraction residues from the tea extract solution, and may be subjected to centrifugation or the like as necessary to remove fine powder. The conditions for centrifugation (e.g., flow rate, rotation speed) may be appropriately selected considering the clarity of the tea beverage finally obtained or the like. To perform centrifugation, it is desirable to cool the extract solution to about 5 to 40°C. Centrifugation after cooling increases the clarity of the tea beverage finally obtained.

In a preferred embodiment, the tea beverage of the present invention has a pH (hydrogen ion concentration index) of 3.5 to 7.5. In general, when the pH of a tea beverage decreases, the liquid color of the tea beverage may change, and it is preferable that the pH of a tea beverage be in a weakly acidic to neutral pH region. In a more preferred embodiment, the pH of the tea beverage of the present invention is 4.6 to 7.0, and may be 5.2 to 6.5. The pH of the beverage may be adjusted by known methods. For example, the adjustment can be performed by adding a pH adjusting agent such as ascorbic acid, citric acid, potassium carbonate, sodium bicarbonate (sodium hydrogen carbonate), sodium hydroxide, or disodium hydrogen phosphate.

### (Coffee Beverage)

In one embodiment of the present invention, the beverage of the present invention can be a coffee beverage. As used herein, a "coffee beverage" refers to a beverage product produced by using a coffee content as a raw material. The type of the product is not particularly limited, but representative examples thereof include "coffee", "coffee beverages", and "coffee-containing soft drinks", which are defined in the "Fair Competition Rules on Labelling of Coffee Beverages and the like" approved in 1977 in Japan. Even in the beverages obtained by using a coffee content as a raw material, the beverages with milk solids of 3.0% by mass or more are taken as "milk beverages" according to the "Fair Competition Rules on Labelling of Milk for Beverage" in Japan, but these beverages are included in the coffee beverage in the present invention.

The roasted coffee beans for use as a raw material for the coffee extract solution of the present invention are not particularly limited. The roasted coffee beans may be prepared by a method such as a direct roasting, hot air, semi-hot air, charcoal-firing, far-infrared radiation, microwave, or superheated water vapor method, using an apparatus such as a horizontal (lateral) drum type, vertical (longitudinal) drum type, vertical rotating ball type, fluidized bed type, or pressurized type apparatus to finish to the roasting degree in accordance with the predetermined purpose, depending on the species of coffee bean. One example of preferred embodiments includes roasted coffee beans roasted to an index value, as measured by an Agtron color meter (Agtron value), of about 35 to 60, preferably about 45 to 50 . The species of coffee beans are not limited, and both of the arabica and robusta species can be used, but the robusta species is an example of preferred embodiments because it is easy to adjust the concentration of the coffee-specific diterpene compounds (cafestol and kahweol) to a specific range in the present invention. The coffee extract solution is obtained by subjecting the roasted coffee beans described above to extraction by a conventional method using a water-soluble solvent such as warm water.

As used herein, the "coffee content" refers to a solution containing a component derived from a coffee bean, and examples thereof include a coffee extract solution, that is a solution obtained by subjecting roasted and ground coffee beans to extraction using water, warm water, or the like. Examples of the coffee content also include a solution prepared by adjusting a coffee extract, that is a concentrated coffee extract solution, an instant coffee that is a dried coffee extract solution, or the like to an appropriate amount with water, warm water, or the like.

The coffee beverage of the present invention may contain caffeine, or may not contain caffeine.

Milk in a milk-containing coffee beverage refers to an ingredient added to impart milk flavor and milk taste to a coffee beverage. Examples of the raw material that provides non-fat milk solid contents include raw milk, cow's milk, certified milk, partially skimmed milk, processed milk, cream, concentrated milk, sugar-free condensed milk, powdered whole milk, powdered cream, powdered butter milk, powdered conditioned milk, skimmed milk, concentrated whey, skimmed concentrated milk, sugar-added skimmed condensed milk, powdered skimmed milk, and powdered whey. Examples of the raw materials that provide milk fat include raw milk, milk, certified milk, partially skimmed milk, processed milk, cream, concentrated milk, sugar-free condensed milk, powdered whole milk, powdered cream, powdered butter milk, and powdered conditioned milk. Instead of some or all of the milk fats, vegetable fats and oils may be used. Examples of the vegetable fats and oils include vegetable fats and oils such as rapeseed oil, cured rapeseed oil, rice oil, soybean oil, corn oil, safflower oil, sunflower oil, cottonseed oil, sesame oil, olive oil, palm oil, palm kernel oil, coconut oil, cured coconut oil, and hydrogenated oils thereof, and ester exchange oils with a mixture of one or more of them.

### (Fruit Juice-Containing Beverage)

In a preferred embodiment of the present invention, the beverage of the present invention can be a fruit juice-containing beverage. In the present invention, the "fruit juice" includes not only those obtained by juicing fruits, but also vegetable juice obtained by juicing vegetables. The fruit used as the raw material for fruit juice may be those commonly used in the art, and is not particularly limited. Examples thereof include citrus fruits (orange, satsuma, Citrus unshiu, navel, ponkan, summer tangerine, lemon, grapefruit, lime, hassaku, iyo, yuzu, camcam, hirami lemon, kabosu, mandarin, tangerine, temple orange, tangelo, calamansi, and the like), strawberry, raspberry, blueberry, blackberry, black currant, cherry, apple, grape, pomegranate, kiwi, muscat, peach, pineapple, guava, banana, passion fruit, mango, acerola, prune, papaya, passion fruit, Japanese apricot, Japanese pear, apricot, lychee, melon, watermelon, pear, persimmon, loquat, fig, and plum. The fruit used as a raw material of the fruit juice is preferably citrus fruits. The fruit juice may be one using the above fruit either alone or in combination of two or more. When two or more fruits are used in combination, the ratio of each fruit (fruit juice) can be appropriately adjusted as necessary and is not particularly limited.

The raw material for the vegetable juice obtained by juicing a vegetable may be those commonly used in the art, and is not particularly limited. Examples thereof include carrot, onion, broccoli, Japanese radish, cabbage, Brussels sprouts, leaves of Brussels sprouts, celery, spinach, green pepper, asparagus, young barley leaves, spring chrysanthemum, Chinese cabbage, Indian mustard, Boston lettuce, Japanese mustard spinach, bok choy, ashitaba, sweet potato, potato, tomato, molokheiya, paprika, cresson, parsley, celery, Japanese honewort, lettuce, radish, kale, leaves of Brussels sprouts, perilla, eggplant, oriental radish, kidney beans, pumpkin, burdock, green onion, ginger, garlic, Chinese chive, leaf mustard, cauliflower, corn, snap peas, okra, turnip, cucumber, Kohlrabi, gourd, zucchini, sponge gourd, bean sprouts, and various sprouts. The vegetable juice may be one using the above vegetable either alone or in combination of two or more. When two or more vegetables are used in combination, the ratio of each vegetable (vegetable juice) can be appropriately adjusted as necessary and is not particularly limited.

Fruit juice can be obtained by juicing a fruit or vegetable as a raw material by known methods in the art. Examples of the known methods include a method of pressing and juicing a raw material by using a hydraulic press machine, a roller squeezer or an inline squeezer; a method of crushing and juicing a raw material using a pulper finisher or the like; and a method of crushing a raw material using a crusher or the like, then juicing the crushed raw material using an extractor or the like, where the raw material is pretreated as necessary by washing, sterilizing, peeling, removing skins, seeds or the like, blanching, crushing, straining, or the like. Those pressed (juiced) by these methods may optionally be further subjected to an enzymatic treatment with pectinase, cellulase, or the like, to a juicer, or to sterilization. Furthermore, the fruit juice may be concentrated as needed. Examples of the concentration method here include concentration by normal heating, decompression concentration, low temperature concentration, vacuum concentration, freeze concentration, and reverse osmosis concentration. The type of fruit juice may be either a straight fruit juice that uses a fruit juice obtained by juicing a fruit as it is or a concentrated fruit juice in which the fruit juice is concentrated. In addition, the fruit juice used may be an opaque fruit juice, or may be a clear fruit juice.

The properties of the fruit juice are not particularly limited and may be, for example, any of a liquid, a gel, a paste (pseudo-solid), a semi-solid, or a solid. The fruit juice may contain other ingredients (e.g., small amounts of salt, spices, food additives, and the like) as needed.

The preparation of the fruit juice can be omitted by obtaining a commercial product. As commercially available products, a straight fruit/vegetable juice, a mixed fruit juice/vegetable juice, a paste, a puree, a concentrated puree, and the like are available. The straight fruit juice/vegetable juice here represents a juice itself obtained by juicing a single fruit or vegetable. The mixed fruit juice/vegetable juice here represents a juice obtained by juicing a plurality of fruits and/or vegetables.

The "straight juice" here refers to a juice specified by the JAS standards, that is, a juice itself obtained by juicing a fruit, or a juice in which only the ingredients allowed by the JAS standards have been added. The straight fruit juice and the fruit juice from concentrate are those obtained by juicing a fruit and optionally adding the ingredients allowed by the JAS standards, and those obtained by concentrating the same in a predetermined proportion. Examples of the ingredients allowed by the JAS standards include antioxidants such as vitamin C (including L-ascorbic acid and sodium L-ascorbate), sugar, honey, and natural flavors. Further examples include a fruit juice in which additives other than those allowed by the JAS standards, e.g., an acidulant such as citric acid or sodium citrate, a pH adjusting agent, an enzyme, a stabilizing agent such as pectin, saccharides other than sugar, and a synthetic flavor, are used.

In a preferred embodiment, the beverage of the present invention contains a citrus fruit juice. Examples of the citrus fruits can include plants belonging to the genus Citrus (Citrus), the genus Fortunella, the genus Poncirus, or the like of the family Rutaceae, the subfamily Aurantioideae, the tribe Citreae (Aurantieae). Preferred examples thereof include citrus, hirami lemon, yuzu, sudachi, lime, lemon, kabosu, orange, and grapefruit, and particularly preferably lemon, orange, and grapefruit. The type of fruit juice may be either a straight fruit juice that uses a fruit juice obtained by juicing a fruit as it is or a concentrated fruit juice in which the fruit juice is concentrated. In addition, the fruit juice may be an opaque fruit juice, or may be a clear fruit juice. The amount of fruit juice added is not particularly limited, and is preferably 0.01 to 50%, more preferably 0.1 to 40%, and further preferably 0.5 to 30%, or may be 1% or more or 3% or more, on a straight fruit juice basis.

### (Others)

The beverage of the present invention can be suitably used, for example, as a food for obtaining functions of chitosan, tea extract, or the like. Specifically, the beverage of the present invention can be a food for specified health uses, a food with nutritional functions, a food for the elderly, a food for special uses, or a functional food.

The beverage may be prepared by a known method for producing a food composition, for example, by mixing chitosan of the present invention in a predetermined amount with a raw material of the known food composition. The beverage may also be prepared by adding chitosan of the present invention in a predetermined amount to a ready-made known food composition. The addition timing and addition method are not particularly limited.

The beverage of the present invention can be ingested orally in an appropriate manner depending on the form thereof. It should be noted that "ingestion" herein is used as encompassing any mode of ingestion, taking, or drinking. The ingestion amount of the beverage of the present invention may be appropriately set depending on the form, the method of ingestion, the intended use, and the age, weight, and symptom of the person who ingests the beverage.

The beverage of the present invention may contain, in addition to the above-described catechin, a sweetener, an acidulant, a fruit juice, various additives, or the like to the extent that the effect of the present invention is not hindered. Examples of the various additives include a flavor, a vitamin, a pigment, an antioxidant, an emulsifier, a preservative, a seasoning, an extract, a pH adjusting agent, and a quality stabilizing agent.

The beverage of the present invention may be a carbonated beverage containing carbon dioxide, but it is preferable that the beverage of the present invention is a non-carbonated beverage. The beverage of the present invention can be an alcoholic beverage, but it is preferable that the beverage of the present invention is a non-alcoholic beverage. The "non-alcoholic beverage" herein refers to a beverage in which the alcohol concentration (ethanol concentration) in the beverage is less than 1%.

The soluble solid content of the beverage of the present invention can be evaluated by degrees Brix obtained by using a saccharimeter, a refractometer, or the like. The degree Brix is a value obtained by converting the refractive index measured at 20°C to the mass/mass percent of a sucrose solution based on the International Commission for Uniform Methods of Sugar Analysis (ICUMSA) conversion table, and represents the soluble solid content in a solution. The units are indicated by "°Bx", "%" or "degree". In a preferred embodiment, the beverage of the present invention has degrees Brix of 0 to 20%, but more preferably the beverage has degrees Brix of 10% or less, and may have degrees Brix of 3% or less or 2% or less.

Since cloudiness is unlikely to occur over time in the beverage of the present invention, the present invention is suitably applied to a relatively light-colored beverage. The color of the beverage can be evaluated by using a light transmittance color-difference meter, or the like. The transparency of the beverage can be quantified, for example, by a known method of measuring the turbidity of a liquid. In a preferred embodiment, for example in an oolong tea beverage, the absorbance at 680nm as the turbidity may be 0.3 or less, or may be 0.01 or more and 0.2 or less.

In a preferred embodiment, the beverage of the present invention is a beverage in which precipitation hardly occurs even after long-term storage at room temperature. In other words, precipitation over time is prevented in the beverage of the present invention, and the appearance of the beverage can be maintained uniformly over a long period of time, and thus the beverage of the present invention is suitably provided as a containerized beverage. The "containerized beverage" herein refers to a beverage that is contained in a resin container such as a polyethylene terephthalate container, a can, a bottle, a paper container, or the like. Since the beverage of the present invention can prevent the precipitation in beverage over time, it is preferable in one embodiment that the beverage of the present invention be a containerized beverage filled in a transparent container.

The containerized beverage of the present invention is produced, for example, by heat-sterilizing a formulated solution obtained in a formulation step, and then filling into a container, in order to store the beverage at room temperature for a long time. The heat sterilization treatment may be performed as prescribed in the Food Sanitation Act. For example, the heat sterilization treatment may be performed by UHT sterilization (e.g., holding the formulated solution at 100 to 150°C for 1 second to several tens of seconds) in a resin container such as a polyethylene terephthalate bottle.

In one aspect, the present invention can be understood as a method for producing a beverage. As described above, the beverage of the present invention contains chitosan, and the content of chitosan is adjusted to a specific range. For example, a method for producing the beverage of the present invention may include a step of adding predetermined ingredients to prepare a beverage, a step of adjusting pH of the beverage, a step of filling the beverage into a container, and the like. In a preferred embodiment, it is preferable that a solution containing chitosan and vitamin C be prepared and then the solution be added to produce a containerized beverage.

The containerized beverage of the present invention can be produced by known methods in the art. Those skilled in the art can appropriately set conditions for an adding method, an optionally sterilization method, and a container filling method.

In yet another aspect, the present invention can be understood as a method for preventing a beverage from clouding. As described above, the beverage of the present invention contains vitamin C and chitosan, and the content of the chitosan is adjusted to a specific range. Specifically, the present invention includes a method for preventing a beverage from clouding, including adding specific amounts of chitosan and vitamin C to the beverage.

### EXAMPLES

Hereinafter, the present invention will be described in more detail with reference to specific experimental examples, but the present invention is not limited to the following specific examples. Unless otherwise stated herein, concentrations or the like are based on weight, and numerical ranges are described as including the endpoints thereof.

### Experiment 1: Production and Evaluation of Oolong Tea Beverage

The tendency of aggregation of chitosan in a tea beverage was evaluated by adding chitosan and vitamin C to a semi-fermented tea, oolong tea.

Specifically, a solution containing chitosan (polymerization degree: 4000, average molecular weight: 650 kDa) and vitamin C (NACALAI TESQUE, INC.) was prepared, and then this solution was added to an oolong tea extract solution to produce a containerized oolong tea beverage containing chitosan and vitamin C at the concentrations shown in the table below (pH of the beverage: about 5).

The presence or absence of cloudiness in the containerized oolong tea beverage was then visually evaluated at a stage where the produced containerized oolong tea beverage was stirred in a vortex mixer and then allowed to stand still at room temperature for 1 hour.

### [Table 1]

**Table 1. Evaluation results of oolong tea beverage**

| | | | | | | | | | | |
|---|---|---|---|---|---|---|---|---|---|---|
| Chitosan concentration (ppm) | 150 | 150 | 150 | 150 | 100 | 100 | 100 | 80 | 80 | 60 |
| Vitamin C concentration (ppm) | 150 | 633 | 1233 | 1983 | 585 | 985 | 1485 | 851 | 1251 | 1061 |
| Cloudiness | Present | Present | Present | Present | Present | Present | Present | Present | Present | **Present** |
| | | | | | | | | | | |
| Chitosan concentration (ppm) | 80 | 80 | 60 | 60 | 50 | 50 | 1 | | | |
| Vitamin C concentration (ppm) | 80 | 531 | 60 | 521 | 50 | 516 | 491 | | | |
| Cloudiness | Absent | Absent | Absent | Absent | Absent | Absent | Absent | | | |

The results of evaluating occurrence of cloudiness for the prepared containerized oolong tea beverage are shown in the table. When the chitosan concentration was 85 ppm or less and the vitamin C concentration was 700 ppm or less, no cloudiness was observed, and it was confirmed that cloudiness caused by chitosan in the oolong tea beverage was effectively prevented.

### Experiment 2: Production and Evaluation of Green Tea Beverage

The tendency of aggregation of chitosan in a tea beverage was evaluated by adding chitosan and vitamin C to a non-fermented tea, green tea.

Specifically, a solution containing chitosan (polymerization degree: 4000, average molecular weight: 650 kDa) and vitamin C (NACALAI TESQUE, Inc.) was prepared, and then this solution was added to a green tea extract solution to produce a containerized green tea beverage containing chitosan and vitamin C at the concentrations shown in the table below (pH of the beverage: about 5).

The presence or absence of cloudiness was then visually evaluated for the containerized green tea beverage at a stage where the produced containerized green tea beverage was stirred with a vortex mixer and then allowed to stand still at room temperature for 1 hour.

### [Table 2]

**Table 2. Evaluation results of green tea beverage**

| | | | | | | | | |
|---|---|---|---|---|---|---|---|---|
| Chitosan concentration (ppm) | 150 | 150 | 100 | 100 | 50 | | | |
| Vitamin C concentration (ppm) | 150 | 396 | 100 | 348 | 738 | | | |
| Cloudiness | Present | Present | Present | Present | Present | | | |
| | | | | | | | | |
| Chitosan concentration (ppm). | 50 | 30 | 30 | 30 | 10 | 10 | 10 | 1 |
| Vitamin C concentration (ppm) | 50 | 30 | 273 | 393 | 10 | 258 | 348 | 251 |
| Cloudiness | Absent | Absent | Absent | Absent | Absent | Absent | Absent | Absent |

The results of evaluating occurrence of cloudiness for the prepared containerized green tea beverage are shown in the table. When the chitosan concentration was 85 ppm or less and the vitamin C concentration was 700 ppm or less, no cloudiness was observed, and it was confirmed that cloudiness caused by chitosan in the green tea beverage was effectively prevented.

### Experiment 3: Production and Evaluation of Black Tea Beverage

The tendency of aggregation of chitosan in a black tea beverage was evaluated by adding chitosan and vitamin C to a fermented tea, black tea.

Specifically, a solution containing chitosan (polymerization degree: 4000, average molecular weight: 650 kDa) and vitamin C (NACALAI TESQUE, Inc.) was prepared, and then this solution was added to a black tea extract solution to produce a containerized black tea beverage containing chitosan and vitamin C at the concentrations shown in the table below (pH of the beverage: about 5).

The presence or absence of cloudiness of the containerized black tea beverage was then visually evaluated at a stage where the produced containerized black tea beverage was stirred in a vortex mixer and then allowed to stand still at room temperature for 1 hour.

### [Table 3]

**Table 3. Evaluation results of black tea beverage**

| | | | | | | | | | |
|---|---|---|---|---|---|---|---|---|---|
| Chitosan concentration (ppm) | 150 | 150 | 100 | 100 | | | | | |
| Vitamin C concentration (ppm) | 150 | 750 | 100 | 500 | | | | | |
| Cloudiness | Present | Present | Present | Present | | | | | |
| | | | | | | | | | |
| Chitosan concentration (ppm) | 50 | 50 | 30 | 30 | 30 | 10 | 10 | 10 | 1 |
| Vitamin C concentration (ppm) | 50 | 250 | 30 | 150 | 300 | 10 | 50 | 100 | 151 |
| Cloudiness | Absent | Absent | Absent | Absent | Absent | Absent | Absent | Absent | Absent |

The results of evaluating occurrence of cloudiness for the prepared containerized tea beverage are shown in the table. When the chitosan concentration was 85 ppm or less and the vitamin C concentration was 700 ppm or less, no cloudiness was observed, and it was confirmed that cloudiness caused by chitosan in the tea beverage was effectively prevented.

### Experiment 4: Production and Evaluation of Coffee Beverage

The tendency of aggregation of chitosan in a coffee beverage was evaluated by adding chitosan and vitamin C to coffee.

Specifically, a solution containing chitosan (polymerization degree: 4000, average molecular weight: 650 kDa) and vitamin C (NACALAI TESQUE, Inc.) was prepared, and then this solution was added to a coffee extract solution to produce a containerized coffee beverage containing chitosan and vitamin C at the concentrations shown in the table below (pH of the beverage: about 5).

The presence or absence of cloudiness of the containerized coffee beverage was then visually evaluated at a stage where the produced containerized beverage was stirred in a vortex mixer and then allowed to stand still at room temperature for 1 hour.

### [Table 4]

**Table 4. Evaluation results of coffee beverage**

| | | | | | | | | | |
|---|---|---|---|---|---|---|---|---|---|
| Chitosan concentration (ppm) | 150 | 150 | 100 | 100 | | | | | |
| Vitamin C concentration (ppm) | 150 | 750 | 100 | 500 | | | | | |
| Cloudiness | Present | Present | Present | Present | | | | | |
| | | | | | | | | | |
| Chitosan concentration (ppm) | 50 | 50 | 30 | 30 | 30 | 10 | 10 | 10 | 1 |
| Vitamin C concentration (ppm) | 50 | 500 | 30 | 150 | 300 | 10 | 50 | 100 | 10 |
| Cloudiness | Absent | Absent | Absent | Absent | Absent | Absent | Absent | Absent | Absent |

The results of evaluating occurrence of cloudiness for the prepared containerized coffee beverage are shown in the table. When the chitosan concentration was 85 ppm or less and the vitamin C concentration was 700 ppm or less, no cloudiness was observed, and it was confirmed that cloudiness caused by chitosan in the beverage was effectively prevented.

### Experiment 5: Production and Evaluation of Fruit Juice-Containing Beverage

The tendency of aggregation of chitosan in a fruit juice-containing beverage was evaluated by adding chitosan and vitamin C to a fruit juice-containing beverage.

Specifically, a solution containing chitosan (polymerization degree: 4000, average molecular weight: 650 kDa) and vitamin C (NACALAI TESQUE, Inc.) was prepared, and then this solution was added to a fruit juice beverage to produce a containerized fruit juice-containing beverage containing chitosan and vitamin C at the concentrations shown in the table below. As the fruit juice, commercially available orange juice (straight type, fruit juice percentage: 100%, vitamin C concentration: 320 ppm) was used, and by diluting it with water, a fruit-juice containing beverage with fruit juice percentage of 10% or 30% (pH of the beverage: about 3.5) was produced.

The presence or absence of cloudiness was then visually evaluated for the containerized fruit juice-containing beverage at a stage where the produced containerized beverage was stirred in a vortex mixer and then allowed to stand still at room temperature for 1 hour.

### [Table 5-1]

**Table 5-1. Evaluation results of fruit juice-containing beverage (fruit juice percentage 30%)**

| | | | | | | | | | | | | |
|---|---|---|---|---|---|---|---|---|---|---|---|---|
| Chitosan concentration (ppm) | 100 | 100 | 100 | 80 | | | | | | | | |
| Vitamin C concentration (ppm) | 196 | 596 | 1096 | 888 | | | | | | | | |
| Cloudiness | Present | **Present** | **Present** | **Present** | | | | | | | | |
| | | | | | | | | | | | | |
| chitosan concentration (ppm) | 60 | 80 | 50 | 50 | 50 | 30 | 30 | 30 | 10 | 10 | 10 | 1 |
| Vitamin C concentration (ppm) | 168 | 488 | 141 | 341 | 581 | 123 | 243 | 393 | 105 | 146 | 195 | 106 |
| Cloudiness | Absent | Absent | Absent | Absent | Absent | Absent | Absent | Absent | Absent | Absent | Absent | Absent |

### [Table 5-2]

**Table 5-2. Evaluation results of fruit juice-containing beverage (fruit juice percentage 10%)**

| | | | | | | | |
|---|---|---|---|---|---|---|---|
| Chitosan concentration (ppm) | 100 | 100 | 100 | | | | |
| Vitamin C concentration (ppm) | 132 | 532 | 1032 | | | | |
| Cloudiness | Present | Present | Present | | | | |
| | | | | | | | |
| Chitosan concentration (ppm) | 30 | 30 | 30 | 10 | 10 | 10 | 1 |
| Vitamin C concentration (ppm) | 61 | 181 | 331 | 42 | 82 | 132 | 33 |
| Cloudiness | Absent | Absent | Absent | Absent | Absent | Absent | Absent |

The results of evaluating occurrence of cloudiness for the prepared containerized fruit juice-containing beverage are shown in the table. When the chitosan concentration was 85 ppm or less and the vitamin C concentration was 700 ppm or less, no cloudiness was observed, and it was confirmed that cloudiness caused by chitosan in the fruit juice-containing beverage was effectively prevented. It is considered that the occurred cloudiness was aggregates of complex polysaccharides such as fruit-derived pectin with chitosan.

## Claims

1. A tea beverage, coffee beverage or fruit juice-containing beverage, comprising 1 to 75 ppm of chitosan and 1 to 650 ppm of vitamin C, wherein a molecular weight of the chitosan is 100 to 1000 kDa.

2. The beverage according to claim 1, comprising the chitosan at a concentration of 1 to 60 ppm.

3. The beverage according to claim 1 or 2, wherein the beverage is a containerized beverage.

4. A method for producing a tea beverage, coffee beverage or fruit juice-containing beverage, comprising adding 1 to 75 ppm of chitosan and 1 to 650 ppm of vitamin C, wherein a molecular weight of the chitosan is 100 to 1000 kDa.

5. A method for preventing a tea beverage, coffee beverage or fruit juice-containing beverage from clouding, the method comprising adding 1 to 75 ppm of chitosan and 1 to 650 ppm of vitamin C, wherein a molecular weight of the chitosan is 100 to 1000 kDa.

## Patentansprüche

1. Ein Teegetränk, Kaffeegetränk oder Fruchtsaft enthaltendes Getränk, umfassend 1 bis 75 ppm Chitosan und 1 bis 650 ppm Vitamin C, wobei ein Molekulargewicht des Chitosans 100 bis 1000 kDa beträgt.

2. Das Getränk nach Anspruch 1, umfassend das Chitosan in einer Konzentration von 1 bis 60 ppm.

3. Das Getränk nach Anspruch 1 oder 2, wobei das Getränk ein containerisiertes Getränk ist.

4. Ein Verfahren zur Herstellung eines Teegetränks, Kaffeegetränks oder Fruchtsaft enthaltenden Getränks, umfassend Zugabe von 1 bis 75 ppm Chitosan und 1 bis 650 ppm Vitamin C, wobei ein Molekulargewicht des Chitosans 100 bis 1000 kDa beträgt.

5. Ein Verfahren zum Verhindern der Trübung eines Teegetränks, Kaffeegetränks oder Fruchtsaft enthaltenden Getränks, wobei das Verfahren Zugabe von 1 bis 75 ppm Chitosan und 1 bis 650 ppm Vitamin C umfasst, wobei ein Molekulargewicht des Chitosans 100 bis 1000 kDa beträgt.

## Revendications

1. Boisson de thé, boisson de café ou boisson contenant du jus de fruit, comprenant 1 à 75 ppm de chitosane et 1 à 650 ppm de vitamine C, dans laquelle un poids moléculaire du chitosane est de 100 à 1000 kDa.

2. Boisson selon la revendication 1, comprenant le chitosane à une concentration de 1 à 60 ppm.

3. Boisson selon la revendication 1 ou 2, dans laquelle la boisson est une boisson remplie dans un conteneur.

4. Méthode de production d'une boisson de thé, d'une boisson de café ou d'une boisson contenant du jus de fruit, comprenant l'addition de 1 à 75 ppm de chitosane et 1 à 650 ppm de vitamine C, dans laquelle un poids moléculaire du chitosane est de 100 à 1000 kDa.

5. Méthode pour éviter qu'une boisson de thé, une boisson de café ou une boisson contenant du jus de fruit ne se trouble, la méthode comprenant l'addition de 1 à 75 ppm de chitosane et 1 à 650 ppm de vitamine C, dans laquelle un poids moléculaire du chitosane est de 100 à 1000 kDa.
